# EUROPEAN PATENT SPECIFICATION

(11) **EP 1 557 143 B1**
(45) Date of publication and mention of the grant of the patent: **14.11.2007**
(21) Application number: 05250286.1
(22) Date of filing: 20.01.2005
(51) Int. Cl.: A61F 2/44

(54) **Intervertebral disc prosthesis**
Zwischenwirbelprothese
Prothèse intervertébrale

(30) Priority: 23.01.2004 GB 0401489
(43) Date of publication of application: 27.07.2005
(73) Proprietor: Corin Limited, Cirencester Gloucestershire GL7 1YJ (GB)
(72) Inventor: Collins, Simon Nicholas, Gloucester, GL8 8NT (GB); Taylor, Sarah Catherine, Gloucester GL8 8HR (GB); Choksey, Munchi Soli, Fillongley CV7 8EA (GB)
(74) Representative: McLeish, Nicholas Alistair Maxwell

(56) References cited:
- EP-A- 0 560 140
- EP-A- 1 344 506
- WO-A-00/23015
- WO-A-02/085261
- WO-A-20/04080349
- DE-U1- 20 310 432
- US-A1- 2003 167 091

## Description

### Field of the Invention

The present invention relates to an intervertebral disc prosthesis.

### Background of the Invention

A large number of people suffer from back pain and, in particular, in those regions of the neck and the back where the spinal column has a significant freedom of movement. Thus the neck and the lumber region are frequent sites of debilitating pain.

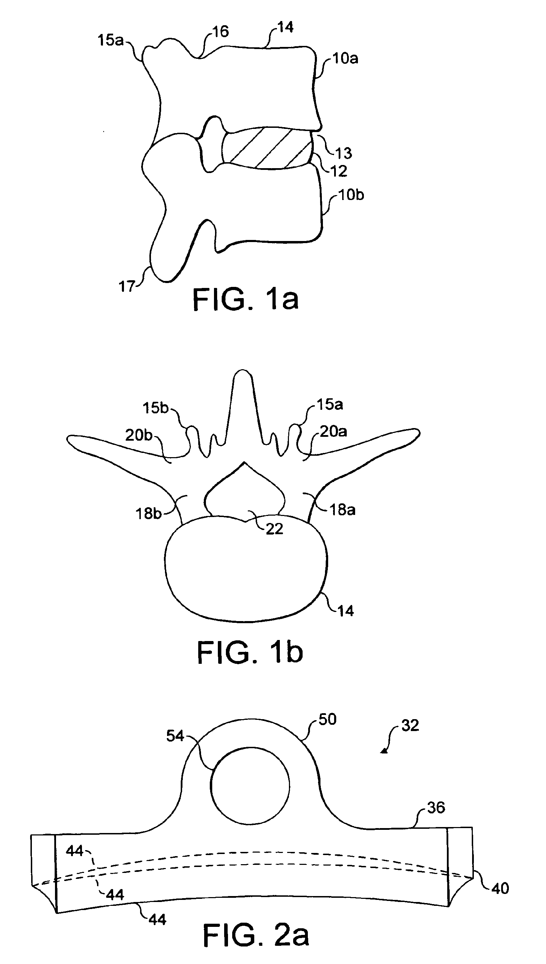

Figure 1a shows two adjacent vertebrae 10a and 10b with an intervertebral disc 12 located in an intervertebral space 13 formed between the two vertebrae 10a and 10b. Each vertebrae consist of a vertebral body 14, which is the anterior, more massive part of the bone that gives strength to the vertebral and supports body weight. A vertebral arch 16 is formed to the posterior of the vertebral body 14 and, as shown in Figure 1b, is formed by right and left pedicles 18a and 18b and right and left lamina 20a and 20b. The pedicles 18a and 18b and lamina 20a and 20b also define a vertebral foramen 22, through which the spinal cord (not shown) passes.

Right and left superior articular processes 15a and 15b project upwardly from the junction of the right and left pedicles 18a and 18b with the right and left laminae 20a and 20b. At the same time right and left inferior articular processes, shown in Figure 1b as 17, project downwardly from the same junction. The superior and inferior articular processes 15 and 17 of adjacent vertebrae 10a and 10b join to form the zygapophysical joint, also known as the facet joint, which permits gliding movements between the two adjacent vertebrae.

Surgery may be performed on the spine for a variety of reasons. These reasons may include when a trauma occurs to the neck or back which results in the rupture of one or more intervertebral discs. Other examples are when herniation or protrusion of an intervertebral disc occurs, for example due to degradation of the disc. If conservative treatment of back pain does not result in an improvement in the condition, or the damage to the disc is unlikely to heal using conservative methods, then spinal surgery may be used in an attempt to correct the problem, alleviate pain and/or maintain the spinal column in a form suitable for protecting the spinal cord.

Previous surgical methods employed to treat a degenerated, ruptured or fractured disc include the removal of a portion, or all of, the disc. Following the removal of the disc, the adjacent vertebrae may be fused using a variety of methods, for example rods screws, plates, bone grafts or fusion cages.

However, the use of spinal fusion affects the biomechanics of the spine and may cause other parts of the spine to degenerate more rapidly than anticipated or usual due to changes in the motion of, or loading applied to, other areas of the spine. For example, removal of an intervertebral disc from the intervertebral space between the C3-C4 vertebrae may accelerate deterioration of the C2-C3 or C4-C5 segments. One reason for this accelerated deterioration is that spinal fusion results in a loss of the rotational or translational movement between the fused vertebrae which is possible when a natural disc is present.

More recently alternative procedures have been proposed for corrective spinal surgery. These alternatives allow some degree of movement between the vertebrae after the removal of degenerated, ruptured or fractured discs.

One procedure is disclosed in US 5,865,846 where an intervertebral disc prosthesis is used which has relatively stiff concaval/convex elements at least partially surrounding a relatively supple central nucleus portion. Information is obtained regarding the size, shape and nature of the patient's damaged spine using methods such as CT or MRI scans. Once the information has been collated then one or more prosthetic disc units are constructed and implanted into the patient's spine.

Another procedure is disclosed in WO 00/23015. Here an intervertebral disc prosthesis is described which comprises two components; a ball component which is attached to one vertebrae and a trough component which is attached to an adjacent vertebrae. The trough component includes a generally concave surface with a flat portion running therethrough. When the ball component and the trough component are inserted into the prepared intervertebral space, and are attached to the respective adjacent vertebrae, they inter-engage to permit a degree of rotational and translational motion of the vertebrae.

Although these intervertebral prostheses allow for some rotational motion and, in the case of WO 00/23015 some translational motion, this motion may be restricted, thereby affecting the biomechanics of the spinal column. Once again this may lead to excessive stresses on other parts of the spine and/or on certain areas of the prosthesis, thereby causing enhanced wear.

It is therefore desired to provide an intervertebral disc prosthesis which provides enhanced rotational and translational movement.

CA 2 482 403 discloses an intervertebral prosthesis comprising two components with one convex and one concave part spherical articulating surface wherein the articulating surfaces extend substantially entirely across that part of each component which is received withing the intervertebral disc space.

The two components further have attachment surfaces having screw holes for fastening the prosthesis to the vertebral body.

WO 2004/080349 which falls under the provision of Art. 54(3) EPC, discloses an intervertebral prosthesis comprising two components with two saddle shaped articulating surfaces wherein the articulating surfaces extend substantially entirely across that part of each component which is received withing the intervertebral disc space.

The two components further have attachment surfaces being configured such that they are capable of nesting with each other.

### Summary of the Invention

According to the present invention there is provided an intervertebral prosthesis as claimed in the claims.

Preferably, the centre of curvature lies on a median plane through the centre of the first and second vertebral bodies.

Providing an intervertebral prosthesis with an attachment surface adapted to nest with the attachment surface of an adjacent intervertebral prosthesis is advantageous since nesting the attachment surfaces optimises use of the limited bone surface available, thereby giving optimal attachment characteristics and allowing for multilevel disc replacements.

The provision of the apertures in the first and second attachment surfaces being spaced from a median plane passing through the centre of the prosthesis by a different amount from the one or more apertures provided in said second attachment surface facilitates nesting of the attachment surfaces. However, a further advantage of situating the apertures in different sagittal planes is that, should the prosthesis require replacement in a revision operation, the attachment surfaces of the revision prosthesis could be provided with apertures lying in different planes to those of the prosthesis being replaced. This would mean that the revision prosthesis could be secured into undamaged bone, i.e. bone not previously used for attachment, to provide a more secure attachment.

Preferably, the apertures provided in both said first and second attachment means are disposed symmetrically with respect to said median plane.

Advantageously, the first and second components further comprise attachment means for attachment to the first and second vertebral bodies.

Preferably, the attachment means is selecting from the group consisting of pegs, plates, posts and screws.

Alternatively or additionally the attachment means includes a surface coating to facilitate bone ingrowth. Preferably, the surface coating is selected from the list comprising Titanium or Cobalt Chrome plasma spray and/or an osteoconductive material such as hydroxyapatite.

Advantageously, the first and second components are formed of biocompatible material.

Preferably, the biocompatible material is selected from the list comprising of Titanium alloy, Cobalt Chrome Stainless Steel, Alumina, Zirconia or any combinations of metal or ceramic biomaterials. Advantageously, at least one of said first and second surfaces is provided with a coating or modified surface layer to enhance lubrication and wear properties. For example Titanium Nitride.

### Brief Description of the Drawings

The preferred embodiments of the invention will now be described, by way of example, with reference to the accompanying drawings, in which:
Figure 1a shows a side view of two adjacent vertebrae within an intervertebral disc therein between;
Figure 1b shows a plan view of a vertebrae;
Figure 2a shows a front view partially in cross-section of a first portion of an intervertebral disc prosthesis in accordance with a preferred embodiment of the present invention;
Figure 2b shows a side view partially in cross-section of the first component of Figure 2a;
Figure 2c shows a perspective view of the first component of Figures 2a and 2b;
Figure 3a shows a front view of a second component of an intervertebral disc prosthesis for use with the first component shown in Figures 2a-c;
Figure 3b shows a perspective view of the second component of Figure 3a;
Figure 3c shows a side view partially in cross-section of the second component of Figures 3a and 3b;
Figure 4 shows a front view of two nested intervertebral disc prostheses comprising first and second components as shown in Figures 2a-c and 3a-c;
Figure 5a shows a posterior perspective view of a further design of an intervertebral prosthesis;
Figure 5b shows an anterior perspective view of the intervertebral prosthesis shown in Figure 5a;
Figure 5c shows a posterior view of the intervertebral prosthesis shown in Figures 5a and 5b;
Figure 6a shows a side view of the intervertebral prosthesis shown in Figures 5a-c;
Figure 6b shows the intervertebral prosthesis of Figure 6a in flexion;
Figure 6c shows the intervertebral prosthesis of Figure 6a in extension;
Figure 7 shows an intervertebral prosthesis further comprising attachment keels;
Figure 8 shows a side view partially in cross-section of an intervertebral disc prosthesis comprising two components in accordance with a further arrangement not being part of the invention;
Figure 9 shows a plan view of one component of the intervertebral disc prosthesis shown in Figure 8 attached to a vertebral body; and
Figures 10a-c show a side view of an intervertebral disc prosthesis according to a further arrangement not being part of the invention.

### Detailed Description of the Drawings

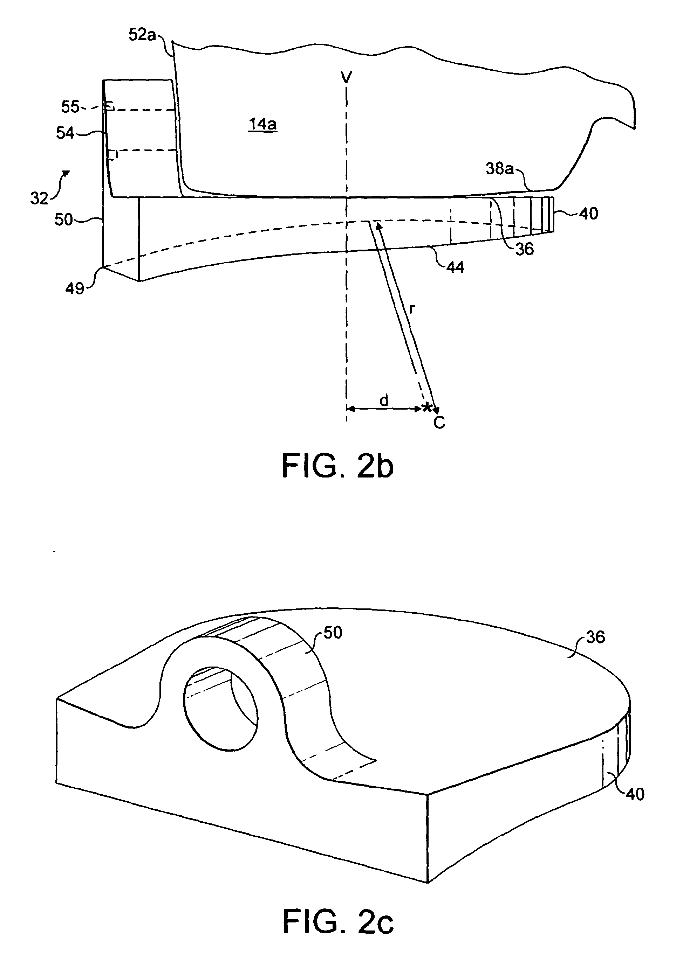

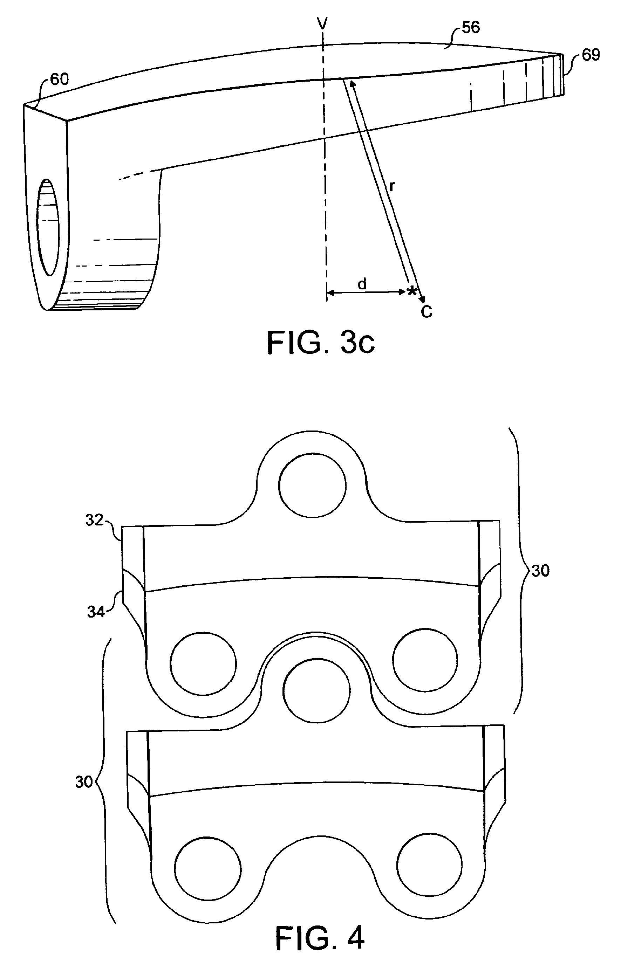

Referring to Figures 2-4 there is shown a first embodiment of an intervertebral disc prosthesis 30 according to the present invention. Prosthesis 30 includes a first prosthetic component 32 and a second prosthetic component 34, which are adapted to be received within at least part of an intervertebral disc space formed between two adjacent vertebral bodies, described subsequently as the superior and inferior vertebral bodies 14a and 14b, the vertebral bodies having vertebral endplates 38a and 38b. The first and second portions 32 and 34 interengage, as shown in Figure 4, to form the prosthesis 30.

Figures 2a-c show the first prosthetic component 32 in more detail. The first prosthetic component 32 comprises a vertebrae engaging surface 36 which is configured to be attached to, or to lie in contact with, the prepared endplate 38a of the superior vertebral body 14a. The vertebrae engaging surface 36 terminates at a posterior edge in a downwardly depending wall 40. The wall 40 in turn merges with a uniformly concave articulating surface 44 defined by a part spherical surface having a single centre of curvature C and a single radius of curvature r, the centre of curvature having a displacement d from a coronal plane v through the centre of the vertebral endplates 38a and 38b. At an anterior edge 49 a flange 50 extends substantially vertically upwardly from the concave articulating surface 44 and is configured to lie in close proximity with an anterior surface 52a of the superior vertebral body 14a.

The flange 50 takes a male form. More specifically, in the embodiment shown in figures 2-6, this form is a curved configuration sized to accommodate an aperture 54. The aperture 54 is sized to allow a fixation means, for example a bone screw, to pass through for attaching the flange 50 to the anterior surface 52a. The aperture 54 may further comprise a countersunk portion 55 to accept the head of the bone screw, thereby allowing the top of the screw to lie flush with a front surface 51 of the flange 50. In other words, the screw head will preferably lie completely below the surface of the flange (50). Advantageously a fixation means will be used in which the screw head is countersunk and in addition clips into recesses provided in the countersunk portion 55 (recesses not shown). An example of such a fixation means is the Cervive^{™} screw, details of which may be found in US 6,613,053 issued on 9 February 2003.

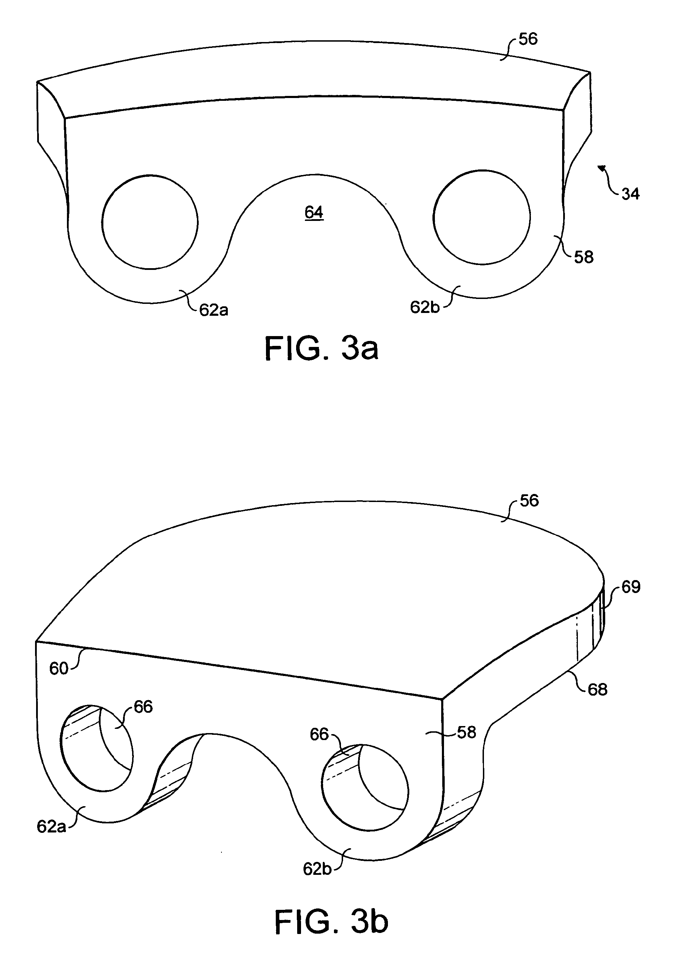

Figures 3a, 3b and 3c show the second prosthetic component 34 in more detail. The second prosthetic component 34 comprises a vertebrae engaging surface 68 which is configured to be attached to, or lie in contact with, the prepared endplate 38b of the inferior vertebral body 14b. The vertebrae engaging surface 68 terminates at a posterior edge in an upwardly extending wall 69. The wall 69 in turn merges with a uniformly convex articulating surface 56, defined by a part spherical surface designed to interengage with the concave articulating surface 44 to allow both rotational and translational motion. The convex articulating surface 56 has the same centre of curvature C, radius of curvature r and displacement d as the concave articulating surface 44. This configuration means that when the prosthesis 30 is located in the intervertebral space it will allow for flexion, extension, lateral bending and rotation of the spine in a manner which approximates as close as possible to normal movement.

The second prosthetic component 34 further comprises a flange 58 which depends substantially vertically downwardly from an anterior edge 60 of the convex articulating surface 56 and is configured to lie in close proximity with an anterior surface 52b of the inferior vertebrae 14b.

The flange 58 takes a female form complementary to that of the male flange 50. More specifically, in this embodiment the female flange 58 comprises two lugs 62a and 62b with a recess 64 formed between the lugs. The recess 64 is shaped to allow the male flange 50 to be at least partially received therein, as shown in Figure 4. Each of the lugs 62a and 62b are sized to accommodate an aperture 66 sized to allow a fixation means, for example a bone screw, to pass through and attach the flange 58 to the anterior surface 52b. An example of a suitable bone screw is the Cervive^{™} screw previously disclosed. As with the aperture 54 of flange 50, the apertures 66 may be countersunk to accept the heads of the screws.

The concave and convex articulating surfaces 44 and 56 are preferably configured such that their centre of curvature C lies at a point in the median plane. The displacement d preferably lies in the range -3mm (anterior of the coronal plane v) and +10mm (posterior of the coronal plane v). Preferably the centre of curvature C is displaced towards the posterior (or rear) of the coronal plane v. Such a displacement allows for enhanced flexion and extension of the spine. Preferably the centre of curvature C lies within the subjacent vertebral body. Preferably the radius of curvature r lies in the range of 20 to 60mm, more preferably 30 to 50 mm and still more preferably 35 to 45 mm.

The configuration of the flanges 50 and 58 as male and female forms means that should two adjacent intervertebral discs require replacement, either at the same time or during a revision operation, then the prosthesis 30 may be fitted with the flanges nesting rather than overlapping, i.e. with a portion of the flange of one component received within a recess formed in the flange of the adjacent component.

Although the flanges 50 and 58 are shown having a curved form it will be understood that they may take other forms, for example with squared corners.

Additionally, although the first prosthetic component 32 is described as having a male flange 50 it will be understood that it is anticipated that the male and female flanges may be interchanged between the two prosthetic components. Thus, the first prosthetic component 32 may be provided with a female flange 58 and the second prosthetic component may be provided with a male flange 50.

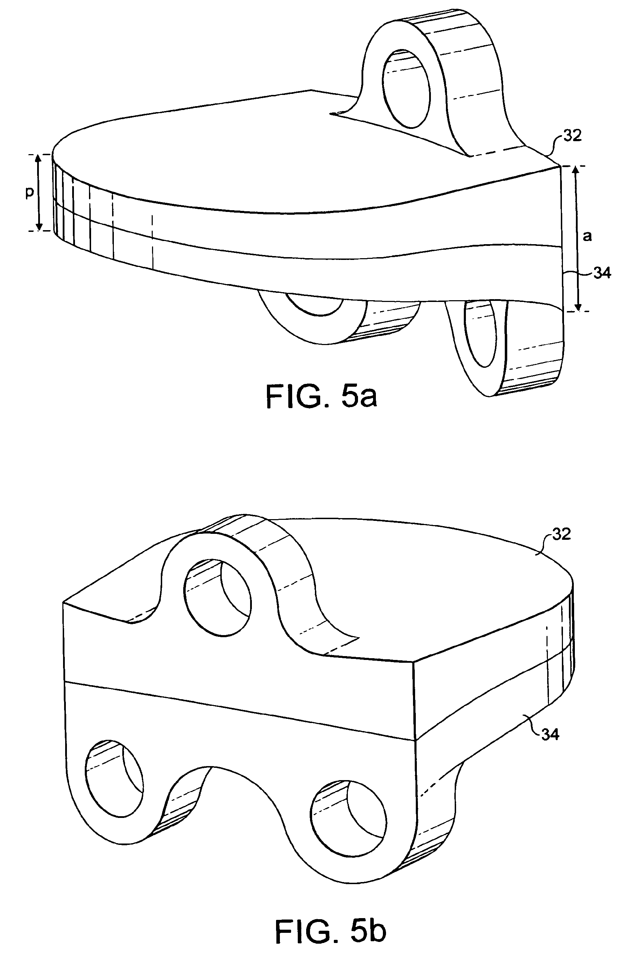

Figure 5a - C show the intervertebral prosthesis 30 as assembled and in particular show the interaction between the concave and convex articulating surfaces 44 and 56. In order to allow lordosis of the spine the intervertebral disc prosthesis preferably occupies a space which is itself in the form of a trapezium, with an anterior dimension a being greater than posterior dimension p as shown in Figure 5a.

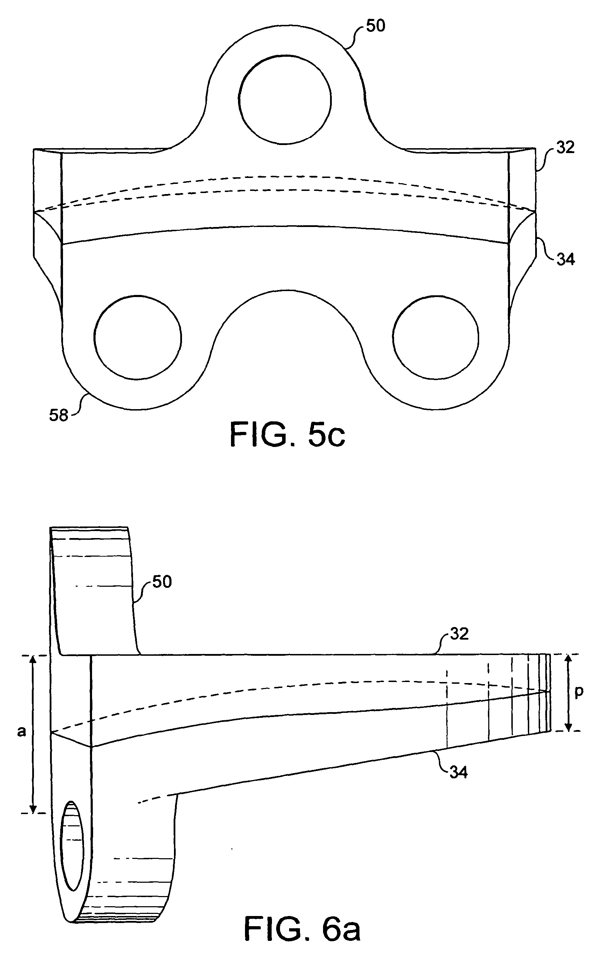

Figure 6a shows a side view of the intervertebral prosthesis 30 and shows in more detail how the first and second prosthetic components 32 and 34 are designed to conform with the natural lordosis.

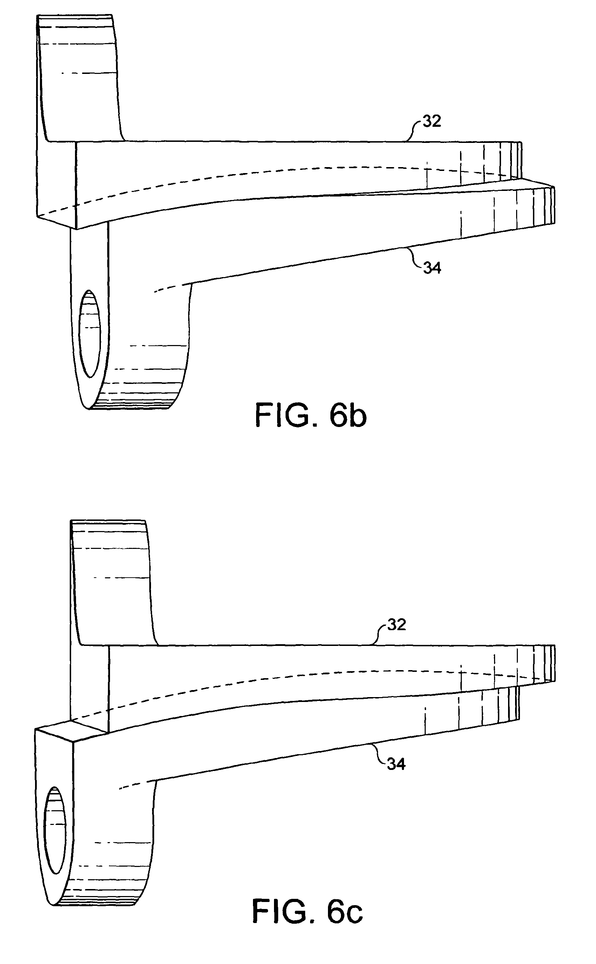

Figures 6b and 6c show the intervertebral prosthesis 30 in flexion and extension respectively.

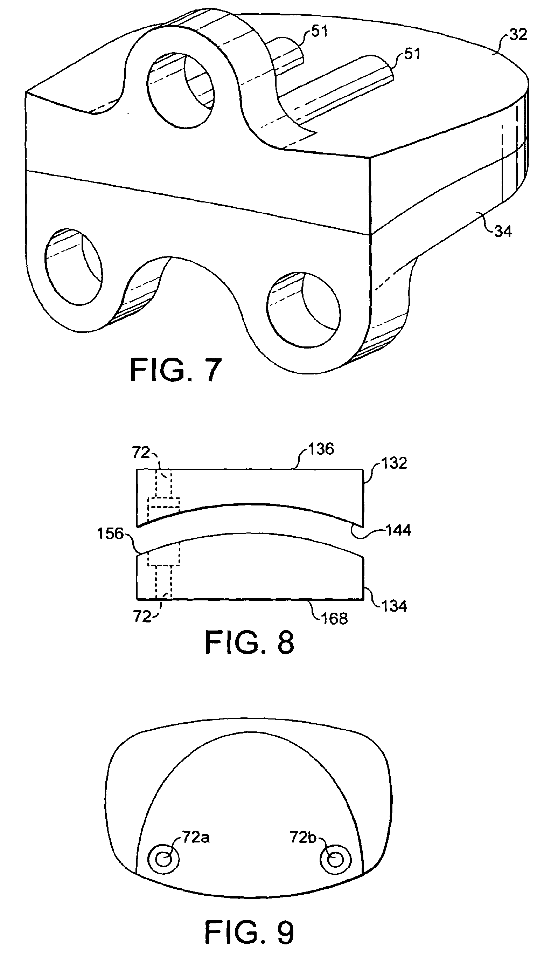

The attachment of the intervertebral prosthesis 30 may be enhanced by the use of one or more keels 51 provided on the vertebrae engaging surfaces 36 and 68 as is shown in Figure 7. The use of such a keel 51 provides a greater surface area in contact with the bone and preferably the contact area of the prosthesis is provided with a surface coating to encourage bone ingrowth. In addition, the or each keel 51 will provide enhanced rotational and translational stability of the intervertebral prosthesis 30. Although the keel 51 is shown as being of semicircular cross-section it will be understood that other suitable cross-sections, for example rectangular or trapezoidal, may be used. Similarly, although the cross-section of each keel 51 is shown as being constant along the length of the keel 51, it will be understood that this is not a requirement, for example the keel 51 may be of increasing or decreasing cross-section along its length. Furthermore, although Figure 7 shows two keels 51, one on either side of the median plane, it will be understood that only one keel may be provided, either on the median plane or offset to one side.

Figures 8 and 9 show an arrangement not being part of the invention, in which the flanges used to attach the prosthesis 30 to the vertebrae bodies 14a and 14b are omitted. For ease of reference the reference numerals for like components are the same as previously used arrangement, with a new stem numeral to indicate a different embodiment. Thus, as before, prosthesis 130 consists of a first prosthetic component 132 and a second prosthetic component 134. Similarly, the first prosthetic component 132 comprises a vertebrae engaging surface 136, which is configured to contact the prepared endplate 38a of a superior vertebrae 14a, and has a uniformly concave articulating surface 144. The concave articulating surface 144 is of part spherical form as described with regard to the previous embodiment.

The second prosthetic component 134 comprises a vertebrae engaging surface 168 adapted to lie in contact with a prepared end surface 38b of an inferior vertebrae 14b and has a uniformly convex articulating surface 156 of part spherical form as described with regard to the previous embodiment.

In place of the flanges 50 and 58, the first and second prosthetic components 132 and 134 comprise countersunk apertures 72a and 72b which are sized to receive bone screws, for example the Cervive^{™} Screw as previously discussed, which may be used to attach the prosthesis 130 to the vertebral bodies 14a and 14b. The apertures 72a and 72b are located anterior to a coronal plane through the centre of the vertebral bodies 14a and 14b to minimise their interference with the articulation between the concave and convex articulating surfaces 144 and 156. As before, the centre of curvature of the concave and convex articulating surfaces 144 and 156 is preferably located on the median plane to the posterior of the same coronal plane.

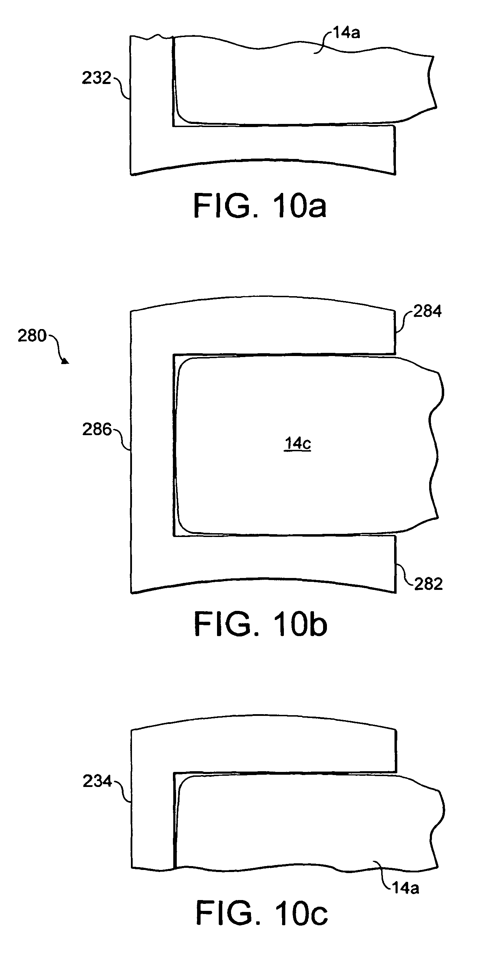

A further arrangement not being part of the present invention is shown in figures 10a-c. In this embodiment a prosthesis 230 is specifically designed for the replacement of two or more adjacent intervertebral discs. In the arrangement shown a three piece prosthesis 230 is provided, the prosthesis 230 comprising a one piece central prosthetic component 280 and two end prosthetic components 232 and 234. The end prosthetic components 232 and 234 correspond to the first prosthetic component 32 and second prosthetic component 34 previously described.

In one configuration the central prosthetic component 280 is configured to fit over the vertebrae intermediate the discs to be replaced, such that an upper prosthetic block 284 lies in the superior intervertebral space and a lower prosthetic block 282 lies in the inferior intervertebral space. The upper prosthetic block 284 is configured substantially similarly to the second prosthetic component 34 described in relation to Figures 3a-c and has a uniformly convex articulating surface 256 as its uppermost surface. At the same time the lower prosthetic block 282 is configured substantially similarly to the first prosthetic component 32 described in relation to Figures 2a-c and has a uniformly concave articulating surface 244 as its lowermost surface.

Rather than individual flanges being provided on each block, as in the first described embodiment, an intermediate portion 286 extends between the first and second prosthetic blocks 284 and 282 such that the upper prosthetic block 284 and lower prosthetic block 282 are held in spaced relation so as to be correctly located in the adjacent intervertebral spaces. Preferably the upper prosthetic block 284 has a vertebral engaging surface 268 and the second prosthetic block 282 has a vertebral engaging surface 236, these surfaces being connected by the intermediate portion 286 which comprises an anterior vertebral engaging surface 288.

Alternatively the central prosthetic component 280 may be configured to replace a damaged vertebrae and in so doing provide articulating surfaces on its upper and lower surfaces, 256 and 244, for interengagement with end prosthetic components 232 and 234.

The surgical procedure used to insert the intervertebral prosthesis is as follows. Firstly, information is found out about the size, shape and nature of the patient's damaged vertebral body, or bodies, using one or more of x-rays, CT scans and MRI scans. In particular, the anterior/posterior and lateral dimensions of the endplates of the vertebral bodies defining the relevant intervertebral disc space(s) are collated. In addition, the height of the relevant intervertebral disc space(s) are ascertained. A correctly sized intervertebral disc prosthesis is then chosen to correlate with the measured dimensions. The intervertebral disc or discs which require replacement are removed and the endplates of the respective vertebrae are then milled or otherwise prepared to receive the vertebral engaging surfaces of the prosthesis.

If a prosthesis is to be used according to the invention shown in Figures 2-4 and 6 - 7, i.e. with flanges, it may also be desirable to remove some of the anterior face of the vertebrae to allow the flanges to be sunk into the anterior face. This preparation may be by milling or any other suitable preparation method. However, it will also be understood that the flanges may sit directly on the anterior face of the vertebral body if desired, in particular if the remaining bone would not provide adequate fixation if part of the anterior face were removed.

The prosthesis is then offered up to the intervertebral space, preferably using a introducer tool. If bone screws are to be used as the primary method of fixation, then the introducer tool preferably includes apertures through which holes can be drilled, or which will allow access to pre-drilled holes, thereby facilitating correct placement of the screws into the vertebral body. The screws, may then be threaded into the vertebrae through the apertures to fix the components to the respective vertebrae. The screws can then be tightened to securely attach the prosthesis to the vertebral body.

Although the attachment of the prosthetic components 32, 34, 132, 134, 232, and 234 to the vertebrae has been described as by the use of bone screws it is understood that other methods of attachment may be used as either a primary or secondary method of attachment. For example, adhesives or staples may also be used. Other examples of fixation methods include the use of teeth, protrusions or webs to bite into the vertebrae to help securement. In addition, a coating may be provided on the vertebrae engaging surface(s) to encourage bone in-growth to provide additional fixation.

The intervertebral prosthesis is preferably made of a biocompatible material such as Cobalt Chrome or titanium. The articulating surfaces may be ceramic on ceramic or metal on metal or other suitable biocompatible materials. If desired the prosthesis may be coated, for example with Titanium Nitrite, to provide a more suitable attachment and/or bearing surface.

## Claims

1. An intervertebral prosthesis (30, 130, 230) adapted to be at least partially received within an intervertebral disc space defined between a first endplate of a first vertebral body and a second endplate of a second, adjacent vertebral body, said prosthesis comprising;
a first component (32,132,232) having an anterior end and opposing posterior end and adapted to be attached to said first vertebral body, said first component comprising a first surface (44, 144) which, in use, is directed towards said second endplate and extends substantially entirely across that part of the first component received within the intervertebral disc space, the first surface having a uniformly concave surface defined by a part spherical surface having a single centre of curvature c and a single radius of curvature r extending from the anterior end of the component to the posterior end of the component with no discontinuities therein, the first component further comprising a first attachment surface (50) depending from the anterior end of the first component end adapted to be attached to an anterior surface of said first vertebral body, said attachment surface having a first configuration and being provided with one or more apertures (54) for the receipt of attachment means with which to secure said first component (32) to said first vertebral body; and
a second component (34, 134, 284) having an anterior end and a posterior end and adapted to be attached to said second vertebral body, said second component comprising a second surface (56, 156) engageable with said first surface to permit articulation of said prosthesis, said second surface (56, 156), in use, being directed towards said first endplate and extending substantially entirely across that part of the second component (34, 134, 284) received within the intervertebral disc space, the second surface having a uniformly convex surface defined by a part spherical surface having the same single centre curvature c and radius of curvature r as the first surface and extending from the anterior end of the second component to the posterior end of the second component with no discontinuities therein, the second component further comprising a second attachment surface (58) depending from the anterior end of the second component and adapted to be attached to an anterior surface of said second vertebral body, said attachment surface having a second configuration and being provided with one or more apertures (66) for the receipt of attachment means with which to secure said second component (34) to said second vertebral body,
said first attachment surface (50) and said second attachment surface (58) being configured such that said first attachment surface is capable of nesting with said second attachment surface, the one or more apertures (54) provided in said first attachment surface (50) being spaced from a median plane passing through the centre of the prosthesis (30) by a different amount from said one or more apertures (66) provided in said second attachment surface (58).

2. An intervertebral prosthesis (30, 130, 230) as claimed in claim 1, wherein one or both of said first or second components is provided with a keel (51).

3. An intervertebral prosthesis (30) as claimed in either claim 1 or claim 2, wherein the apertures (54, 66) provided in both said first and second attachment surfaces (50, 58) are disposed symmetrically with respect to said median plane.

4. An intervertebral prosthesis (30, 130, 230) as claimed in any preceding claim, wherein said first and second components further comprise attachment means for attachment to the first and second vertebral bodies.

5. An intervertebral prosthesis (30, 130, 230) as claimed in any preceding claim, wherein said attachment means is selected from the group consisting of pegs, posts, plates and screws.

6. An intervertebral prosthesis (30, 130, 230) as claimed in any preceding claim, wherein said attachment means includes a surface coating to facilitate bone ingrowth.

7. An intervertebral prosthesis (30, 130, 230) as claimed in claim 1 further comprising a secondary attachment means.

8. An intervertebral prosthesis (30, 130, 230) as claimed in any preceding claim received within said intervertebral disc space, wherein, in use, said centre of curvature c is posterior of a coronal plane through the centre of said first and second vertebral endplates.

9. An intervertebral prosthesis (30, 130, 230) as claimed in any preceding claim, wherein, in use, said centre of curvature c lies on a median plane through the centre of said first and second vertebral bodies.

10. An intervertebral prosthesis (30, 130, 230) as claimed in any preceding claim, wherein said first and second components are formed of biocompatible material.

11. An intervertebral prosthesis (30, 130, 230) as claimed in Claim 10, wherein said biocompatible material is selected from the list comprising of Titanium, Cobalt Chrome, Alumina or Zirconia Alloys or any combination of metallic or ceramic biomaterials.

12. An intervertebral prosthesis (30, 130, 230) as claimed in any preceding claim, wherein at least one of said first (44, 144) and second (56, 156) surfaces is provided with a coating or modified surface layer.

## Patentansprüche

1. Zwischenwirbelprothese (30, 130, 230), die dazu angepasst ist, zumindest teilweise in einem Zwischenwirbelscheibenraum aufgenommen zu werden, der zwischen einer ersten Endplatte eines ersten Wirbelkörpers und einer zweiten Endplatte eines zweiten, benachbarten Wirbelkörpers festgelegt ist, wobei die Prothese umfasst:
einen ersten Bestandteil (32, 132, 232), der ein vorderes Ende und ein gegenüberliegendes, hinteres Ende aufweist und dazu ausgelegt ist, am ersten Wirbelkörper befestigt zu werden, wobei der erste Bestandteil eine erste Fläche (44, 144) umfasst, die im Gebrauch in Richtung der zweiten Endplatte weist und sich im Wesentlichen vollständig über jenen Teil des ersten Bestandteils erstreckt, der im Zwischenwirbelscheibenraum aufgenommen ist, wobei die erste Fläche eine gleichmäßig konkave Fläche aufweist, die mittels einer einen einzigen Krümmungsmittelpunkt c und einen einzigen Krümmungsradius r aufweisenden, teilweise kugelförmigen Fläche festgelegt wird, wobei sie sich ohne Unterbrechung vom vorderen Ende des Bestandteils zum hinteren Ende des Bestandteils erstreckt, wobei der erste Bestandteil ferner eine vom vorderen Ende des ersten Bestandteils abhängende, erste Befestigungsfläche (50) umfasst, die dazu ausgelegt ist, an der vorderen Fläche des ersten Wirbelkörpers befestigt zu werden, wobei die Befestigungsfläche eine erste Konfiguration aufweist und mit einer oder mehreren Öffnungen (54) versehen ist, um Befestigungsmittel aufzunehmen, mittels welcher der erste Bestandteil (32) am ersten Wirbelkörper befestigt wird; und
einen zweiten Bestandteil (34, 134, 234), der ein vorderes Ende und ein hinteres Ende aufweist und dazu ausgelegt ist, am zweiten Wirbelkörper befestigt zu werden, wobei der zweite Bestandteil eine zweite Fläche (56, 156) umfasst, die mit der ersten Fläche in Eingriff bringbar ist, um ein Anlenken der Prothese zu erlauben,
wobei die zweite Fläche (56, 156) im Gebrauch in Richtung der ersten Endplatte weist und sich im Wesentlichen vollständig über jenen Teil des zweiten Bestandteils (34, 134, 234) erstreckt, der im Zwischenwirbelscheibenraum aufgenommen ist, wobei die zweite Fläche eine gleichmäßig konvexe Fläche aufweist, die mittels einer teilweise kugelförmigen Fläche festgelegt wird, die den gleichen, einzigen Krümmungsmittelpunkt c und den gleichen Krümmungsradius r wie die erste Fläche aufweist, und wobei sie sich ohne Unterbrechung vom vorderen Ende des zweiten Bestandteils zum hinteren Ende des zweiten Bestandteils erstreckt, wobei der zweite Bestandteil ferner eine zweite Befestigungsfläche (58) umfasst, die vom vorderen Ende des zweiten Bestandteils abhängt und dazu ausgelegt ist, an der vorderen Fläche des zweiten Wirbelkörpers befestigt zu werden, wobei die Befestigungsfläche eine zweite Konfiguration aufweist und mit einer oder mehreren Öffnungen (66) versehen ist, um Befestigungsmittel aufzunehmen, mittels welcher der zweite Bestandteil (34) am zweiten Wirbelkörper befestigt wird;
wobei die erste Befestigungsfläche (50) und die zweite Befestigungsfläche (58) derart konfiguriert sind, dass die erste Befestigungsfläche in der Lage ist, sich mit der zweiten Befestigungsfläche zu verschachteln, wobei die eine Öffnung oder mehreren Öffnungen (54), die in der ersten Befestigungsfläche (50) vorgesehen ist bzw. sind, von einer durch die Mitte der Prothese (30) hindurchtretenden Mittelebene um einen Betrag beabstandet sind, der sich von demjenigen der einen Öffnung oder mehreren Öffnungen (66) unterscheidet, die in der zweiten Befestigungsfläche (58) vorgesehen ist bzw. sind.

2. Zwischenwirbelprothese (30, 130, 230) gemäß Anspruch 1, bei welcher einer oder beide der ersten oder zweiten Bestandteile mit einem Kiel (51) versehen sind.

3. Zwischenwirbelprothese (30) gemäß Anspruch 1 oder Anspruch 2, bei welcher die sowohl in der ersten als auch in der zweiten Befestigungsfläche (50, 58) vorgesehenen Öffnungen (54, 66) bezüglich der Mittelebene symmetrisch angeordnet sind.

4. Zwischenwirbelprothese (30, 130, 230) gemäß einem der vorstehenden Ansprüche, bei welcher die ersten und zweiten Bestandteile ferner Befestigungsmittel zur Befestigung an den ersten und zweiten Wirbelkörpern umfassen.

5. Zwischenwirbelprothese (30, 130, 230) gemäß einem der vorstehenden Ansprüche, bei welcher die Befestigungsmittel aus der Gruppe ausgewählt sind, die aus Stiften, Pfosten, Platten und Schrauben gebildet ist.

6. Zwischenwirbelprothese (30, 130, 230) gemäß einem der vorstehenden Ansprüche, bei welcher die Befestigungsmittel einen Flächenüberzug einschließen, um das Einwachsen von Knochen zu erleichtern.

7. Zwischenwirbelprothese (30, 130, 230) gemäß Anspruch 1, die ferner zusätzliche Befestigungsmittel umfasst.

8. Zwischenwirbelprothese (30, 130, 230) gemäß einem der vorstehenden Ansprüche, die im Zwischenwirbelscheibenraum aufgenommen ist, wobei sich der Krümmungsmittelpunkt c im Gebrauch hinter einer durch die Mitte der ersten und zweiten Wirbelendplatten verlaufenden Koronalebene befindet.

9. Zwischenwirbelprothese (30, 130, 230) gemäß einem der vorstehenden Ansprüche, bei welcher sich der Krümmungsmittelpunkt c im Gebrauch auf einer durch die Mitte der ersten und zweiten Wirbelkörper verlaufenden Mittelebene befindet.

10. Zwischenwirbelprothese (30, 130, 230) gemäß einem der vorstehenden Ansprüche, bei welcher die ersten und zweiten Bestandteile aus einem biokompatiblen Material gebildet sind.

11. Zwischenwirbelprothese (30, 130, 230) gemäß Anspruch 10, bei welcher das biokompatible Material aus der Liste ausgewählt ist, welche Titan, Kobaltchrom, Aluminiumoxid- oder Zirkonoxidlegierungen oder jede beliebige Kombination von metallischen oder keramischen Biomaterialien umfasst.

12. Zwischenwirbelprothese (30, 130, 230) gemäß einem der vorstehenden Ansprüche, bei welcher die ersten Flächen (44, 144) und/oder die zweiten Flächen (56, 156) mit einem Überzug oder einer flächenmodifizierten Schicht versehen sind.

## Revendications

1. Prothèse intervertébrale (30, 130, 230) conçue pour être au moins partiellement reçue dans un espace interdiscal défini entre un premier plateau vertébral d'un premier corps vertébral et un second plateau vertébral d'un second corps vertébral adjacent, ladite prothèse comprenant :
un premier composant (32, 132, 232) présentant une extrémité antérieure et une extrémité postérieure opposée et conçu pour être fixé audit premier corps vertébral, ledit premier composant comprenant une première surface (44, 144) qui, en utilisation, est dirigée vers ledit second plateau vertébral et s'étend globalement entièrement au travers de cette partie du premier composant reçu au sein de l'espace interdiscal, la première surface présentant une surface uniformément concave définie par une surface en partie sphérique ayant un seul centre de courbure c et un seul rayon de courbure r s'étendant de l'extrémité antérieure du composant jusqu'à l'extrémité postérieure du composant sans aucune discontinuité dans celle-ci, le premier composant comprenant en outre une première surface de fixation (50) pendant à partir de l'extrémité antérieure de la première extrémité de composant conçue pour être fixée à une surface antérieure dudit premier corps vertébral, ladite surface de fixation présentant une première configuration et étant dotée d'une ou plusieurs ouvertures (54) destinées à la réception du moyen de fixation avec lequel ledit premier composant (32) est fixé audit premier corps vertébral, et
un second composant (34, 134, 284) présentant une extrémité antérieure et une extrémité postérieure et étant conçu pour être fixé audit second corps vertébral, ledit second composant comprenant une seconde surface (56, 156) pouvant être engagée avec ladite première surface pour permettre l'articulation de ladite prothèse, ladite seconde surface (56, 156), en utilisation, étant dirigée vers ledit premier plateau vertébral et s'étendant globalement entièrement au travers de la partie du second composant (34, 134, 284) reçue au sein de l'espace interdiscal, la seconde surface présentant une surface uniformément convexe définie par une surface en partie sphérique ayant le même centre de courbure unique c et le même rayon de courbure unique r que la première surface et s'étendant de l'extrémité antérieure du second composant jusqu'à l'extrémité postérieure du second composant sans aucune discontinuité dans celle-ci, le second composant comprenant en outre une seconde surface de fixation (58) pendant à partir de l'extrémité antérieure du second composant et conçue pour être fixée à une surface antérieure dudit second corps vertébral, ladite surface de fixation présentant une seconde configuration et étant dotée d'une ou plusieurs ouvertures (66) destinées à la réception d'un moyen de fixation avec lequel ledit second composant (34) est fixé audit second corps vertébral,
ladite première surface de fixation (50) et ladite seconde surface de fixation (58) étant configurées de telle sorte que ladite première surface de fixation peut s'emboîter avec ladite seconde surface de fixation, les une ou plusieurs ouvertures (54) étant prévues dans ladite première surface de fixation (50) qui est espacée d'un plan médian passant par le centre de la prothèse (30) d'une quantité différente par rapport auxdites une ou plusieurs ouvertures (66) prévues dans ladite seconde surface de fixation (58).

2. Prothèse intervertébrale (30, 130, 230) selon la revendication 1, dans laquelle l'un ou les deux desdits premier ou second composants est/sont doté(s) d'une quille (51).

3. Prothèse intervertébrale (30) selon la revendication 1 ou la revendication 2, dans laquelle les ouvertures (54, 66) prévues dans à la fois lesdites première et seconde surfaces de fixation (50, 58) sont disposées de façon symétrique par rapport audit plan médian.

4. Prothèse intervertébrale (30, 130, 230) selon l'une quelconque des revendications précédentes, dans laquelle lesdits premier et second composants comprennent en outre un moyen de fixation destiné à la fixation des premier et second corps vertébraux.

5. Prothèse intervertébrale (30, 130, 230) selon l'une quelconque des revendications précédentes, dans laquelle ledit moyen de fixation est sélectionné à partir du groupe constitué de chevilles, de montants, de plaques et de vis.

6. Prothèse intervertébrale (30, 130, 230) selon l'une quelconque des revendications précédentes, dans laquelle ledit moyen de fixation comprend un revêtement de surface afin de faciliter la repousse osseuse.

7. Prothèse intervertébrale (30, 130, 230) selon la revendication 1, comprenant en outre un moyen de fixation secondaire.

8. Prothèse intervertébrale (30, 130, 230) selon l'une quelconque des revendications précédentes, reçue dans ledit espace intersomatique vertébral, dans laquelle, en utilisation, ledit centre de courbure c est postérieur par rapport à un plan coronal passant par le centre desdits premier et second plateaux vertébraux.

9. Prothèse intervertébrale (30, 130, 230) selon l'une quelconque des revendications précédentes, dans laquelle, en utilisation, ledit centre de courbure c repose sur un plan médian passant par le centre desdits premier et second corps vertébraux.

10. Prothèse intervertébrale (30, 130, 230) selon l'une quelconque des revendications précédentes, dans laquelle lesdits premier et second composants sont formés d'un matériau biocompatible.

11. Prothèse intervertébrale (30, 130, 230) selon la revendication 10, dans laquelle ledit matériau biocompatible est sélectionné à partir de la liste comprenant des alliages de titane, de cobalt-chrome, d'alumine ou de zirconium ou toute combinaison de biomatériaux métalliques ou céramiques.

12. Prothèse intervertébrale (30, 130, 230) selon l'une quelconque des revendications précédentes, dans laquelle au moins une desdites première (44, 144) et seconde (56, 156) surfaces est dotée d'une couche de revêtement ou d'une couche de surface modifiée.
